(19) Europäisches Patentamt
European Patent Office
Office européen des brevets

(11) Veröffentlichungsnummer: **0 065 131**
**A2**

(12) **EUROPÄISCHE PATENTANMELDUNG**

(21) Anmeldenummer: **82103516.9**

(51) Int. Cl.[3]: **C 07 D 233/90**, A 01 N 43/50

(22) Anmeldetag: **26.04.82**

(30) Priorität: **07.05.81 DE 3118146**

(43) Veröffentlichungstag der Anmeldung: **24.11.82**
**Patentblatt 82/47**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Beck, Gunther, Dr., Am Mittelberg 19, D-5090 Leverkusen 1 (DE)**
Erfinder: **Heitzer, Helmut, Dr., Höhenstrasse 84, D-5090 Leverkusen 3 (DE)**
Erfinder: **Eue, Ludwig, Dr., Paul-Klee-Strasse 36, D-5090 Leverkusen 1 (DE)**
Erfinder: **Schmidt, Robert, Im Waldwinkel 110, D-5060 Bergisch-Gladbach 2 (DE)**
Erfinder: **Lürssen, Klaus, Dr., August-Kierspel-Strasse 89, D-5060 Bergisch-Gladbach 2 (DE)**

(54) **Dihalogenierte Imidazolcarbonsäure-alkoxyamide, Verfahren zu ihrer Herstellung und ihre Verwendung als Herbizide.**

(57) Imidazolcarbonsäure-Alkoxyamide der Formel

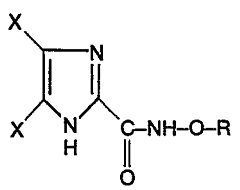

(I)

in welcher
X für Chlor oder Brom steht und
R für Alkyl steht,
besitzen eine sehr gute herbizide Wirksamkeit und sind außerdem als Defoliants und Desiccants geeignet. Die Stoffe der Formel (I) lassen sich nach mehreren Verfahren herstellen.

EP 0 065 131 A2

BAYER AKTIENGESELLSCHAFT 5090 Leverkusen, Bayerwerk

Zentralbereich
Patente, Marken und Lizenzen Dü-by-c
Ib

# Dihalogenierte Imidazolcarbonsäure-alkoxyamide, Verfahren zu ihrer Herstellung und ihre Verwendung als Herbizide

Die vorliegende Erfindung betrifft neue dihalogenierte Imidazolcarbonsäure-alkoxyamide, mehrere Verfahren zu deren Herstellung sowie ihre Verwendung als Herbizide.

Es ist bereits bekannt geworden, daß bestimmte 4,5-Dichlorimidazol-2-carbonsäure-Derivate herbizide Eigenschaften besitzen (vgl. DE-A 2 610 527). So kann z.B. das 4,5-Dichlorimidazol-2-carbonsäure-tert.-butylamid zur Bekämpfung von Unkraut eingesetzt werden. Dieser Stoff ist jedoch nicht immer ausreichend wirksam und in seiner Selektivität nicht immer ganz befriedigend.

Es wurden nun neue dihalogenierte Imidazolcarbonsäure-alkoxyamide der Formel

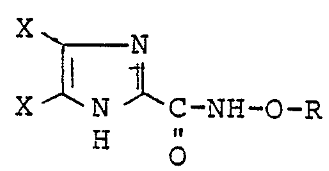

(I)

in welcher

X für Chlor oder Brom steht und

R für Alkyl steht,

gefunden.

Weiterhin wurde gefunden, daß man dihalogenierte Imidazolcarbonsäure-alkoxyamide der Formel (I) erhält, wenn man

a) das dimere Keten der Formel

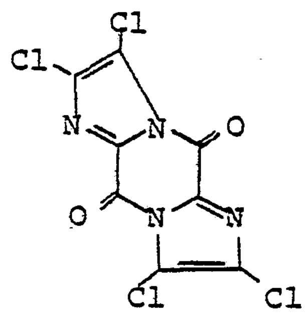

(II)

mit Alkoxyaminen der Formel

$$H_2N-OR \qquad (III)$$

in welcher

R die oben angegebene Bedeutung hat,

gegebenenfalls in Gegenwart eines Verdünnungsmittels sowie gegebenenfalls in Gegenwart eines Katalysators umsetzt,

oder

b) Imidazol-2-carbonsäure-alkoxyamide der Formel

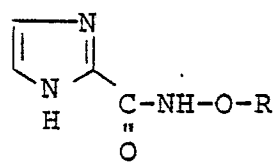

(IV)

in welcher

R die oben angegebene Bedeutung hat,

mit mindestens der zur Dihalogenierung stöchiometrisch erforderlichen Menge an Chlorierungs- oder Bromierungsmittel in Gegenwart eines unter den Reaktionsbedingungen inerten Verdünnungsmittels sowie gegebenenfalls in Gegenwart eines säurebindenden Mittels umsetzt.

Schließlich wurde gefunden, daß die neuen dihalogenierten Imidazolcarbonsäure-alkoxyamide der Formel (I) starke herbizide, insbesondere auch selektiv-herbizide Eigenschaften besitzen. Außerdem eignen sie sich als Defoliants und Desiccants.

Überraschenderweise zeigen die erfindungsgemäßen dihalo-

genierten Imidazolcarbonsäure-alkoxyamide der Formel (I) bei sehr guter Unkrautwirkung insbesondere bessere Möglichkeiten zum Einsatz als selektive Unkrautbekämpfungsmittel in wichtigen Kulturen als das aus dem Stand der Technik bekannte 4,5-Dichlorimidazol-2-carbonsäure-tert.-butylamid, welches ein konstitutionell ähnlicher, hochaktiver Wirkstoff gleicher Wirkungsart ist.

Ferner lassen sich die erfindungsgemäßen Stoffe sehr gut als Entblätterungsmittel (Defoliants) und als Blätteraustrocknungsmittel (Desiccants) verwenden.

Die erfindungsgemäßen dihalogenierten Imidazolcarbonsäure-alkoxyamide sind durch die Formel (I) allgemein definiert. In dieser Formel steht X für Chlor oder Brom. R steht vorzugsweise für geradkettiges oder verzweigtes Alkyl mit 1 bis 6 Kohlenstoffatomen.

Verwendet man bei der Variante (a) des erfindungsgemäßen Verfahrens das dimere Keten der Formel (II) und n-Propoxy-amin als Ausgangsstoffe, so kann der Verlauf der Umsetzung durch das folgende Formelschema wiedergegeben werden:

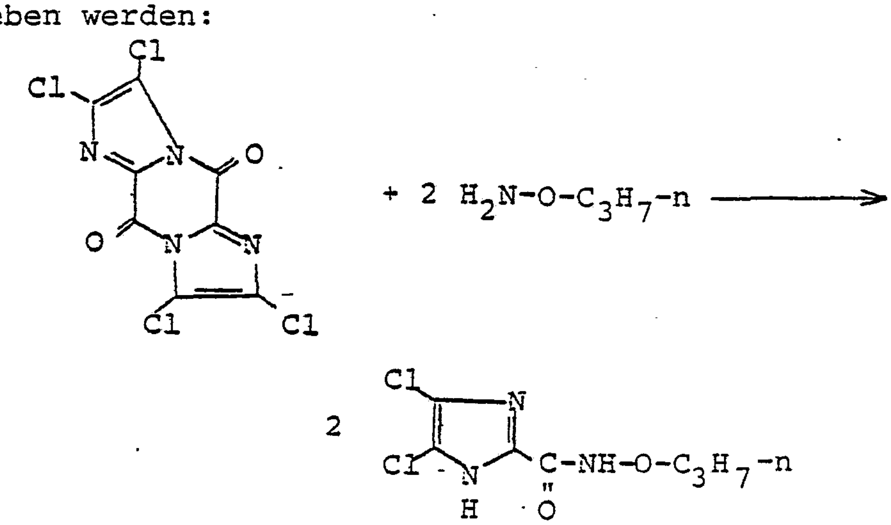

Verwendet man bei der Variante (b) des erfindungsgemäßen Verfahrens Imidazol-2-carbonsäure-N-ethoxyamid und Brom als Ausgangsstoffe, so kann der Verlauf der Umsetzung durch das folgende Formelschema wiedergegeben werden:

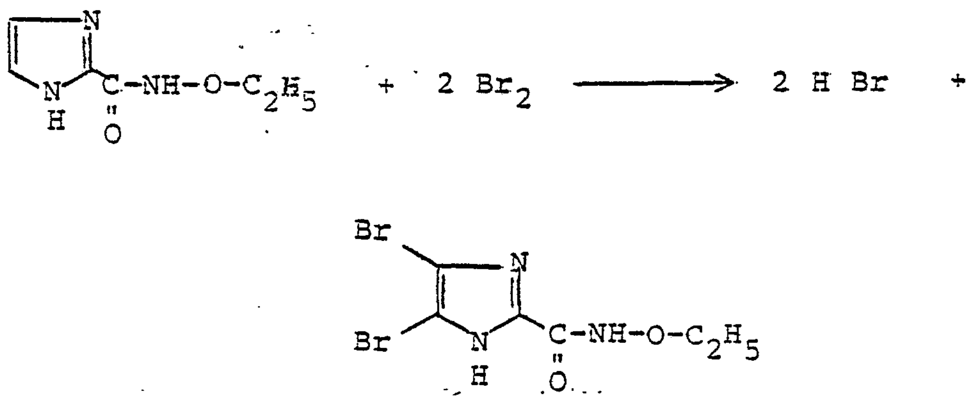


Das bei dem erfindungsgemäßen Verfahren (a) als Ausgangsstoff benötigte dimere Keten der Formel (II) ist bekannt (vgl. DE-A 26 34 053).

Die bei dem erfindungsgemäßen Verfahren (a) weiterhin als Ausgangsstoffe benötigten Alkoxyamine sind durch die Formel (III) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden.

Die Alkoxyamine der Formel (III) sind bekannt oder lassen sich in einfacher Weise nach im Prinzip bekannten Verfahren herstellen (vgl. Bulletin de l'Academie Polonaise

des Sciences, Sêrie des sciences chimiques Vol. XXII, Nr. 3, Seite 195 (1974) und Rocz. Chem. 1974, 48, Seite 139).

Als Verdünnungsmittel kommen bei der Umsetzung nach dem Verfahren (a) sowohl die als Reaktionskomponenten verwendeten Alkoxyamine der Formel (III) selbst, - sofern sie in flüssigem Zustand vorliegen oder bei niedrigen Temperaturen schmelzen -, als auch inerte organische Solventien in Frage. Hierzu gehören vorzugsweise chlorierte Kohlenwasserstoffe, wie Methylenchlorid und Chloroform, sowie Ether, wie Diethylether, Tetrahydrofuran und Dioxan. Ferner können auch Alkohole mit 1 bis 4 Kohlenstoffatomen, wie Methanol oder Isopropanol, oder auch Wasser als Verdünnungsmittel fungieren.

Als Katalysatoren können bei dem Verfahren (a) starke anorganische Basen, wie Natriumhydroxid oder Kaliumhydroxid, oder Amine, wie Triethylamin oder Triethylendiamin, verwendet werden.

Die Reaktionstemperaturen können bei dem Verfahren (a) innerhalb eines größeren Bereiches variiert werden. Im allgemeinen arbeitet man bei Temperaturen zwischen -50°C und +200°C, vorzugsweise zwischen 0°C und 100°C.

Bei der Durchführung des Verfahrens (a) setzt man auf 1 Mol an dimerem Keten der Formel (II) 2 Mol oder auch einen größeren Überschuß an Alkoxyamin der Formel (III) sowie gegebenenfalls 0,01 bis 1, vorzugsweise 0,1 bis 0,5 Mol an Katalysator ein. Die Isolierung der entstehenden dihalogenierten Imidazol-carbonsäurealkoxyamide der Formel (I) erfolgt nach üblichen Methoden. Im allgemeinen verfährt man in der Weise, daß man nach beendeter Umsetzung das Lösungsmittel und/oder die im Überschuß vorhandene Komponente (III) abdestilliert, den verbleibenden Rückstand mit Wasser verrührt, das dabei kristallin anfallende Produkt abfiltriert und gegebenenfalls umkristallisiert.

Die bei dem erfindungsgemäßen Verfahren (b) als Ausgangsstoffe benötigten Imidazol-2-carbonsäure-alkoxyamide sind durch die Formel (IV) allgemein definiert. In dieser Formel hat R vorzugsweise diejenigen Bedeutungen, die bereits im Zusammenhang mit der Beschreibung der erfindungsgemäßen Stoffe der Formel (I) vorzugsweise für diesen Rest genannt wurden.

Die Imidazol-2-carbonsäure-alkoxyamide der Formel (IV) sind bisher noch nicht bekannt; sie lassen sich jedoch nach üblichen Methoden herstellen. So erhält man diese Stoffe, indem man das dimere Keten der Formel

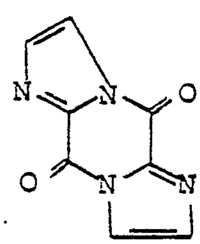

(V)

mit Alkoxyaminen der Formel

$$H_2N-OR \qquad (III)$$

in welcher

R die oben angegebene Bedeutung hat,

gegebenenfalls in Gegenwart eines Verdünnungsmittels sowie gegebenenfalls in Gegenwart eines Katalysators bei Temperaturen zwischen -50°C und +200°C, vorzugsweise zwischen 0°C und +100°C, umsetzt.

Das bei der Herstellung der Imidazol-2-carbonsäure-alkoxyamide der Formel (IV) als Ausgangsstoff benötigte dimere Keten der Formel (V) ist bisher noch nicht beschrieben worden. Es läßt sich jedoch herstellen, indem man Imidazol-2-carbonsäure mit einem Überschuß an Thionylchlorid gegebenenfalls in Gegenwart von Dimethylformamid als Katalysator unter Rückfluß erhitzt.

Die Imidazol-2-carbonsäure ist bekannt (vgl. Acta Chem. Scand. 21, 279 (1967)).

Bei der Herstellung der Imidazol-2-carbonsäure-alkoxyamide der Formel (IV) nach dem oben angegebenen Verfahren kommen als Verdünnungsmittel bzw. als Katalysatoren alle diejenigen Stoffe in Betracht, die in diesem Zusammenhang bei der Beschreibung des erfindungsgemäßen Verfahrens (a) erwähnt wurden. Die Durchführung dieses Verfahrens erfolgt so wie es bei dem erfindungsgemäßen Verfahren (a) beschrieben wurde. Bei der Aufarbeitung verfährt man nach üblichen Methoden. Im allgemeinen geht man so vor, daß man nach beendeter Umsetzung

das Lösungsmittel und/oder die im Überschuß vorhandene Komponente der Formel (III) abdestilliert den verbleibenden Rückstand mit Wasser verrührt, das dabei kristallin anfallende Produkt abtrennt und gegebenenfalls umkristallisiert.

Als Chlorierungs- und Bromierungsmittel können bei dem erfindungsgemäßen Verfahren (b) die Halogene Chlor und Brom, aber auch Chlor angebende Substanzen, wie Sulfurylchlorid, verwendet werden.

Als Säurebindemittel kommen bei dem erfindungsgemäßen Verfahren (b) vorzugsweise Alkali- oder Erdalkalihydroxide, -carbonate oder -hydrogencarbonate, wie z.B. Natriumhydroxid oder Natriumhydrogencarbonat, in Frage.

Als Verdünnungsmittel kommen bei dem erfindungsgemäßen Verfahren (b) alle unter den Reaktionsbedingungen inerten Lösungsmittel in Betracht. Hierzu gehören vorzugsweise Methylenchlorid und Chloroform. Es kann jedoch auch in wäßrigem Medium gegebenenfalls in Gegenwart eines Säurefängers gearbeitet werden.

Die Reaktionstemperaturen können bei dem erfindungsgemäßen Verfahren (b) innerhalb eines größeren Bereiches variiert werden. Im allgemeinen arbeitet man bei Temperaturen zwischen -50°C und +150°C, vorzugsweise zwischen -20°C und +100°C. Dient Wasser als Verdünnungsmittel, so arbeitet man zweckmäßigerweise zwischen 0°C und +50°C.

Bei der Durchführung des erfindungsgemäßen Verfahrens (b) setzt man auf 1 Mol an Imidazol-2-carbonsäure-alkoxyamid der Formel (IV) 2 Mol oder auch einen größeren Überschuß, vorzugsweise 2,5 Mol an Chlorierungs- bzw. Bromierungsmittel, sowie gegebenenfalls zwei oder mehr Äquivalente eines Säurebinders ein. Vorzugsweise verfährt man so, daß man das Imidazol-2-carbonsäure-alkoxyamid der Formel (IV) in einem Verdünnungsmittel vorlegt, dann das Halogenierungsmittel, gegebenenfalls unter Kühlung, in dem Maße zudosiert, daß die gewählte Reaktionstemperatur eingehalten wird. Zur Vervollständigung der Reaktion kann das Reaktionsgemisch nach beendeter Zugabe des Halogenierungsmittels noch weitere 1 bis 5 Stunden auf der gewählten Temperatur gehalten werden. Im Falle der Verwendung von Wasser als Verdünnungsmittel kann der Säurefänger entweder mit dem Imidazol-2-carbonsäure-alkoxyamid der Formel (IV) vorgelegt werden oder aber nach der Zugabe des Halogenierungsmittels zugefügt werden. Auch die gleichzeitige Zugabe von Säurefänger und Halogenierungsmittel ist möglich. In einer weiteren Variante kann auch das Halogenierungsmittel vorgelegt werden und das Imidazol-2-carbonsäure-alkoxyamid der Formel (IV) in Lösung hinzugegeben werden. Die Isolierung der Reaktionsprodukte erfolgt nach üblichen Methoden. Im allgemeinen verfährt man so, daß man bei Verwendung von inerten organischen Solventien als Verdünnungsmittel letztere nach beendeter Umsetzung abzieht und den Rückstand gegebenenfalls reinigt, indem man das Rohprodukt mit wäßriger Salzsäure im Verhältnis 1:1 bei 20°C behandelt. Dabei gehen monohalogenierte Verbindungen und nicht halogeniertes Ausgangsprodukt in Lösung, während das dihalogenierte Produkt in fester Form verbleibt und abfiltriert werden kann. Arbeitet man in Gegenwart von Wasser als Verdünnungsmittel, so erfolgt die Aufarbeitung im allgemeinen dadurch, daß man das Reaktionsgemisch durch Zugabe von wäßriger Alkalibase alkalisch macht, das ausfallende Produkt abtrennt, wäscht und gegebenenfalls umkristallisiert.

Die erfindungsgemäßen Wirkstoffe beeinflussen das Pflanzenwachstum und können deshalb als Defoliants, Desiccants, Krautabtötungsmittel, Keimhemmungsmittel, und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Stoffe eignen sich insbesondere zur selektiven Unkrautbekämpfung, zum Beispiel in Mais, Weizen und Sojabohnen. Sie zeichnen sich durch eine gute Verträglichkeit in wichtigen Kulturen aus und besitzen außerdem ein breites herbizides Wirkungsspektrum. Darüber hinaus können die erfindungsgemäßen Wirkstoffe sehr gut zur Entblätterung von Baumwollpflanzen zur Austrocknung der Blätter von Baumwollpflanzen verwendet werden. Außerdem besitzen die erfindungsgemäßen Stoffe auch eine fungizide Wirksamkeit.

Die Wirkstoffe können in die üblichen Formulierungen übergeführt werden, wie Lösungen, Emulsionen, Suspensionen, Pulver, Schäume, Pasten, Granulate, Wirkstoffimprägnierte Natur- und synthetische Stoffe und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkraubekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

Herstellungsbeispiele

Beispiel 1

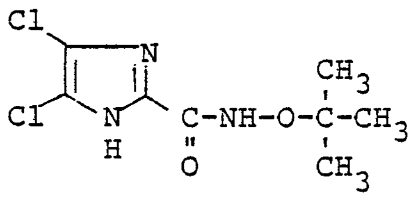

Eine Lösung von 89 g (1 Mol) 1,1-Dimethylethyl-O-hydroxylamin in 2 Liter Dioxan wird bei Raumtemperatur mit 122,3 g (0,375 Mol) der Verbindung der Formel

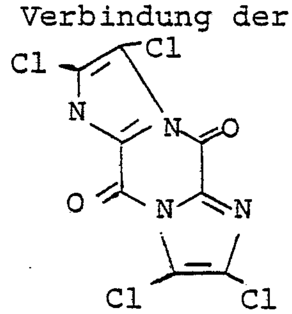

versetzt und eine Stunde unter Rückfluß gerührt. Anschließend zieht man das Lösungsmittel und das überschüssige Amin im Vakuum ab. Der Rückstand wird mit 1 Liter Wasser verrührt; dann wird abgesaugt, mit Wasser nachgewaschen und getrocknet. Man erhält so 181 g (95,8 % der Theorie) 4,5-Dichlorimidazol-2-carbonsäure-N(1,1-dimethylethyloxy)-amid. Schmelzpunkt 204°C (aus Acetonitril).

Nach der im Beispiel 1 angegebenen Methode werden auch die in der folgenden Tabelle formelmäßig angegebenen Stoffe hergestellt:

Tabelle 1

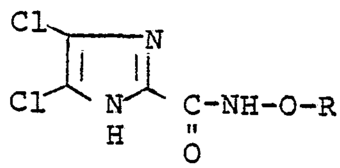

(Ia)

| Beispiel Nr. | R | Schmelzpunkt | umkrist. aus |
|---|---|---|---|
| 2 | $CH_3$ | 245°C | Acetonitril |
| 3 | $C_2H_5$ | 206°C | Acetonitril |
| 4 | iso-$C_3H_7$ | 138°C | Acetonitril |
| 5 | n-$C_4H_9$ | 125°C | Cyclohexan |

Beispiel 6

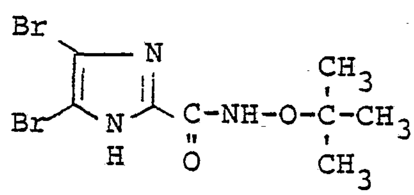

Eine Suspension von 4,71 g (0,026 Mol) Imidazol-2-carbonsäure-N-(tert.-butoxy)-amid in 50 ml Wasser wird bei etwa 10°C im Verlauf von 30 Minuten mit 10,4 g (0,065 Mol) Brom versetzt. Nach zweistündigem Nachrühren bei

Raumtemperatur wird mit 1 N wäßriger Natronlauge auf pH 6 eingestellt, abgesaugt und mit Wasser gewaschen und getrocknet. Ausbeute 4,78 g (53,9 % der Theorie) 4,5-Dibromimidazol-2-carbonsäure-N(1,1-dimethylethyl-oxy)-amid. Schmelzpunkt 219°C (aus wenig Acetonitril).

Herstellung von Ausgangsprodukten

Beispiel (IV-1)

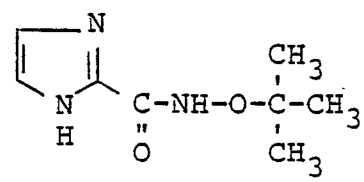

In eine Lösung von 8,9 g (0,1 Mol) 1,1-Dimethylethyl-O-hydroxylamin in 200 ml Dioxan werden bei Raumtemperatur 7,0 g (0,037 Mol) dimeres Keten der Formel

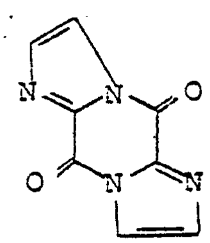

eingerührt und eine Stunde unter Rückfluß gerührt. Anschließend wird im Vakuum einrotiert und der Rückstand mit 200 ml Wasser verrührt. Nach dem Absaugen, Waschen mit Wasser und Umkristallisation des anfallenden Produktes aus Acetonitril erhält man 8,3 g ( 61 % der Theorie) an Imidazol-2-carbonsäure-N-(tert.-butoxy)-amid vom Schmelzpunkt 215°C.

Beispiel V

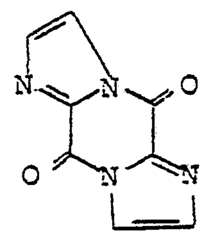

11,2 g (0,1 Mol) Imidazol-2-carbonsäure werden mit 100 ml Thionylchlorid 5 Stunden unter Rückfluß gerührt. Nach dem Erkalten wird abgesaugt, mit etwas Petrolether gewaschen und getrocknet. Man erhält so in fast quantitativer Ausbeute das 5H,10H-diimidazo-/1,2-a:1',2'-d/pyrazin-5,10-dion in Form eines gelben Pulvers. Die Substanz schmilzt noch nicht bei 290°C. Sie ist bei 200-250°C/0,01 Torr unzersetzt in gelben Kristallen sublimierbar.

IR (KBr): 3137, 1735, 1522, 1445, 1387, 1331, 1274, 1161, 1059, 1018, 810, 800, 748, 699, 651 $cm^{-1}$.

In den folgenden Verwendungsbeispielen wurde die nachstehend formelmäßig angegebene Substanz als Vergleichspräparat eingesetzt:

(A) = 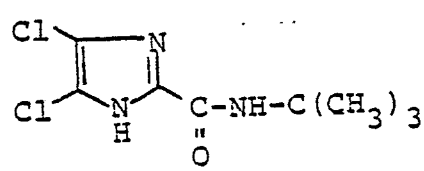 (bekannt aus DE-A 2 610 527)

(4,5-Dichlorimidazol-2-carbonsäure-tert.-butylamid)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (1) und (6) eine bessere Wirksamkeit als die Vergleichssubstanz (A).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigen die erfindungsgemäßen Verbindungen (1), (4) und (6) eine bessere Wirksamkeit als die Vergleichssubstanz (A).

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
+ leichtes Austrocknen der Blätter, geringer Blattfall
++ starkes Austrocknen der Blätter, starker Blattfall
+++ sehr starkes Austrocknen der Blätter, sehr starker Blattfall

In diesem Test zeigt die erfindungsgemäße Verbindung (1) eine sehr starke Wirksamkeit.

Patentansprüche

1. Dihalogenierte Imidazolcarbonsäure-alkoxyamide der Formel

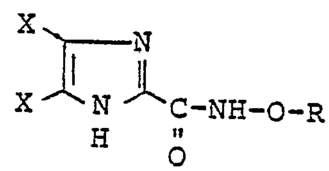

(I)

in welcher

X für Chlor oder Brom steht und

R für Alkyl steht.

2. Dihalogenierte Imidazolcarbonsäure-alkoxyamide der Formel (I), in welcher X für Chlor oder Brom steht und R für geradkettiges oder verzweigtes Alkyl mit 1 bis 6 Kohlenstoffatomen steht.

3. Verfahren zur Herstellung von dihalogenierten Imidazolcarbonsäure-alkoxyamiden der Formel

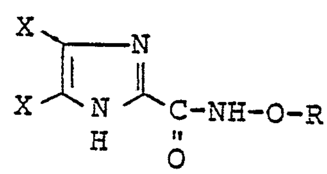

(I)

in welcher

X für Chlor oder Brom steht und

R für Alkyl steht,

dadurch gekennzeichnet, daß man

a) das dimere Keten der Formel

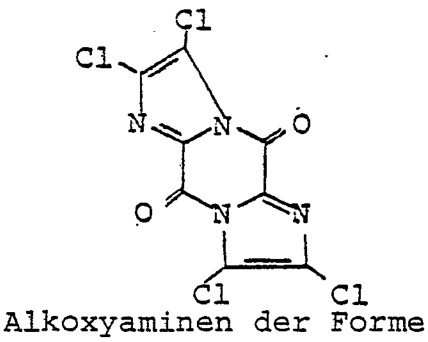

(II)

mit Alkoxyaminen der Formel

$H_2N-OR$ (III)

in welcher

R die oben angegebene Bedeutung hat,

gegebenenfalls in Gegenwart eines Verdünnungsmittels sowie gegebenenfalls in Gegenwart eines Katalysators umsetzt,

oder

b) Imidazol-2-carbonsäure-alkoxyamide der Formel

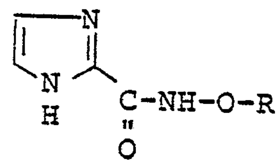

(IV)

in welcher

R die oben angegebene Bedeutung hat,

mit mindestens der zur Dihalogenierung stöchiometrisch erforderlichen Menge an Chlorierungs- oder Bromierungsmittel in Gegenwart eines unter den Reaktionsbedingungen inerten Verdünnungsmittels sowie gegebenenfalls in Gegenwart eines säurebindenden Mittels umsetzt.

4. Herbizide Mittel, gekennzeichnet durch einen Gehalt an mindestens einem dihalogenierten Imidazol-carbonsäure-alkoxyamid der Formel (I).

5. Verfahren zur Bekämpfung von Unkräutern, dadurch gekennzeichnet, daß man dihalogenierte Imidazol-carbonsäure-alkoxyamide der Formel (I) auf Unkräuter und/oder deren Lebensraum ausbringt.

6. Verwendung von dihalogenierten Imidazol-carbonsäure-alkoxyamiden der Formel (I) zur Bekämpfung von Unkräutern bzw. zur Entblätterung von Kulturpflanzen.

7. Verfahren zur Herstellung von herbiziden Mitteln, dadurch gekennzeichnet, daß man dihalogenierte Imidazol-carbonsäure-alkoxyamide der Formel (I) mit Streckmitteln und/oder oberflächenaktiven Stoffen vermischt.

8. Dihalogeniertes Imidazol-carbonsäure-alkoxamid der Formel

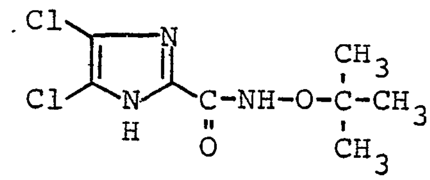

9. Dihalogeniertes Imidazol-carbonsäure-alkoxamid der Formel

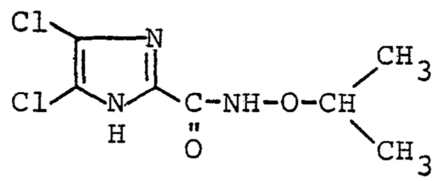

10. Dihalogeniertes Imidazol-carbonsäure-alkoxamid der Formel